# EUROPEAN PATENT APPLICATION

(11) **EP 3 231 862 A1**
(43) Date of publication of application: **18.10.2017**
(21) Application number: 17166711.6
(22) Date of filing: 14.04.2017
(51) Int. Cl.: C12M 1/34, B65D 90/32, C12G 1/02

(54) **CONTROL METHOD AND APPARATUS FOR THE INNER PRESSURE OF A FERMENTATION TANK**

(30) Priority: 15.04.2016 IT UA20162641
(71) Applicant: Noform S.r.l., 30020 Meolo (VE) (IT)
(72) Inventor: Crosato, Stefano, 30020 MEOLO (VE) (IT)
(74) Representative: Citron, Massimiliano

(57) **Abstract**

To reduce the risk of implosion of a tank (MC) capable of fermenting a vegetable product in a pressurized environment, a control method is presented. The tank comprises
- a tube (26) which communicates with the inside of the tank and which descends from the top (12) of the tank down to its base,
- a vacuum-breaker device (36) connected permanently to the tube in proximity of the tank's base to let external air into the tube in case of depression inside the tank.

The method comprises the step of detecting the presence of a column of liquid inside the tube by means of a sensor (32), so as to guarantee the constant operation of the vacuum-breaker device.

## Description

The invention relates to a control method and a control apparatus for the internal pressure of a fermentation tank, in particular to a fermentation tank so controlled and a device that implements the control. The following description will make reference by example to winemaking, sector in which the invention has proven to be particularly effective.

Tank implosion is a known and unfortunately expensive event for the damage it can create, as also reported in the introduction of US4736864. For example, autoclaves, especially for the fermentation of wine or beer, are constructed with a resilient but light structure, to keep costs and weights low. Therefore a small internal depression (40-60 millibars) is enough to destroy them, and a few negative millibars more or less can do the difference between a disaster and a surviving autoclave. Usually the phenomenon is fought with a vacuum-breaker valve arranged on top of the tank, but in reality the problem is solved only partially. As also described in US5071021 column 1 lines 36-40, the internal diaphragm of the vacuum-breaker valve sometimes jams on its own, and is little reliable especially if the internal pressure of the tank, as in autoclaves, is very high. The sticky sugar content of the fermentation vapors, or bees and birds that make the nest there often are causes of malfunction, so the valve needs constant maintenance despite its awkward position.

The main object of the present invention is to improve this state of art.

To this aim, a method is proposed of mounting a vacuum-breaker device or valve connected directly on a vertical pipe that from the tank's top comes down to its base where the device is more conveniently at hand to carry out maintenance and the periodic verification checks of proper operation.

And it is proposed a tank for fermenting a vegetable product in a pressurized environment, the tank comprising
- a pressurizable container,
- a tube which communicates with the inside of the container and which descends from the top of the container to its base,
- a vacuum-breaker device permanently connected to the tube in proximity of the container's base to let outside air into the tube in case of depression inside the container.

By *permanently connected* it is meant that the vacuum-breaker device or valve is never isolated from the vertical tube.

Since the tube communicates with the inside of the tank, it can also be used to read the internal pressure through a pressure gauge present on the vertical tube.

The Applicant has also found out that the condensation of fermentation vapors can form within the vertical tube a column of liquid being even one meter high, and so the liquid column fails the reading of the pressure gauge and prevents the operation of the vacuum-breaker valve.

If someone fills the tank up to overflowing the liquid inside the vertical tube, he will read from the pressure gauge an incorrect value. Not just this: if someone opens the bottom drain valve, by gravity effect the fluid flows out dragging with itself other liquid and the tank implodes, also because the vacuum-breaker valve can not intervene due to the thrust of the liquid column.

In particular, the present invention has as further object the accomplishment of a method to avoid, or at least mitigate, the danger of implosion for a tank, in particular for an autoclave, wherein the internal pressure reaches at least +0.5 relative bars.

More particularly, the present invention has as further object the implementation of a method to avoid, or at least mitigate, the danger of implosion for a tank, in particular for an autoclave, resulting from the presence of liquid in said vertical tube.

Another object is to build a tank to implement said method.

A first aspect of the invention relates to a method for reducing the risk of implosion of a tank capable of fermenting a vegetable product in a pressurized environment, the tank comprising
- a tube which communicates with the inside of the tank and which descends from the top of the tank down to its base,
- a vacuum-breaker device connected permanently to the tube in proximity of the tank's base to let external air into the tube in case of depression inside the tank;
comprising the step of detecting the presence of a column of liquid inside the tube by means of a sensor.

By *sensor* it is meant an artificial sensor of the mechanical and/or electrical type.

The advantage given by the sensor is both to avoid reading an incorrect value of pressure inside the duct, and avoid that, upon ejecting inadvertently from the tube such liquid, the tank implodes; furthermore, to ensure the constant and correct functioning of the anti-vacuum device.

In the following, there are some useful variants of the method, exploitable alone or in combination:
- an electrical signal may be acquired from the sensor, the signal is evaluated by means of an electronic circuit, and in case of presence of liquid a warning device or alarm is activated (in order to warn about the anomaly); and/or
- an electric signal may be acquired from the sensor, the signal is evaluated by means of an electronic circuit, and in case of presence of liquid a discharge means (60) (e.g. a pump or a valve), e.g. mounted on or connected to the tube, is activated automatically for discharging to the outside the liquid accumulated in the tube (in order to solve the anomaly automatically); and/or
- the presence of liquid may be detected/checked at a point at which (optionally) the inner pressure of the tank is detected, and in affirmative case, the liquid is evacuated from the duct to remove the contact with the liquid for said point; and/or
- isolating the tube from the tank before emptying it through said valve or discharge means mounted on the tube (to speed up the evacuation and safeguard the fermentative pressurization of the tank); and/or
- before emptying the tube through the opening of said valve or discharge means, a valve or an opening located on the top part of the tube may be opened, that is, the section connected to the top of the tank and/or the descending and vertical section of the tube that stands higher or nearer to the top of the tank.

Another aspect of the invention relates to a tank for fermenting a vegetable product in a pressurized environment, comprising
- a pressurizable container,
- a tube which communicates with the inside of the container and which descends from the top of the container to its base,
- a vacuum-breaker device permanently connected to the tube in proximity of the container's base to let outside air into the tube in case of depression inside the container;
a sensor for detecting the presence of a liquid column inside the tube.

The device serves to allow the entry of external air into the container if the inner pressure of the container is less than atmospheric pressure, e.g. with a maximum pressure difference equal to 50 millibars.

The device, for constructive simplicity, preferably comprises or is constituted of a valve intended to open if the external pressure is greater than the inner one of the container, preferably with a maximum pressure difference of 50 millibars. Optionally the device also comprises a valve intended to open if the pressure inside the container is greater than a positive pressure threshold. This allows e.g. venting the container and/or absorbing the small oscillations of pressure during the loading operation of the vegetable product.

The vertical tube is connected directly to the top of the container, point at which the container - in use and without any anomalies - should remain empty; and gas accumulates there.

The sensor is able to detect the presence of liquid, preferably of a liquid column, inside the vertical control duct/tube, in order to discard false readings of a possible pressure sensor due to a liquid column inside the control duct or to avoid implosion by siphoning.

In particular, to simplify management and automate the method and the tank with artificial intelligence, the tank for fermenting may comprise a processing unit configured or programmed for
acquiring an electrical signal from the liquid sensor to detect the presence of (a column of) liquid inside the tube, and
driving a second device in case of presence of liquid (e.g. a warning device and/or said valve or discharge means for the tube).

For example, the processing unit may be configured (e.g. by a PCB circuit) or programmed (e.g. via software in a microprocessor) for
acquiring a data from the liquid sensor and executing a function dependent on such data, e.g. reporting an anomaly (presence of liquid) by means of an acoustic or luminous signal emitted from the second device.

In particular, to avoid an implosion caused by a vertical duct/tube full of liquid, the tank may comprise
a first (optional) valve to isolate the duct/pipe from the top of the container, and
a second valve or means for evacuating the duct/pipe,
the processing unit being configured or programmed for closing the first valve (if any) and opening the second valve or the means to evacuate when the liquid sensor detects liquid.

The liquid sensor is preferably placed at the detection point of the pressure sensor, if present.

Preferably the container is an autoclave.

Preferably the vacuum-breaker valve or device is connected to the tube through a fitting with an equal or greater cross-section than the cross-section of the vertical tube, with the advantage of not obstructing the air-passage orifice of the device or valve, avoiding limiting the flow in case of drainage from the vertical tube.

Preferably, for economy, efficiency or ease of programming them, the steps of the methods defined herein are managed and/or executed through a software program and a microprocessor by which the software is executed.

In particular, the program executes some of the above variants through the steps of
- acquiring from a liquid sensor an electrical signal relative to the presence of liquid inside the tube or relative to the liquid level in the tube,
- determining from the electrical signal a data regarding liquid level or presence;
- comparing the data with a liquid threshold,
- depending on the result of the comparison, activating a device, in particularly said second device.

Another variant provides a method to avoid the implosion of a tank capable of fermenting a vegetable product in the pressurized environment, with the steps of:
- acquiring from a sensor an electrical signal relating to the presence or level of a liquid inside said vertical tube;
- determining from the electrical signal a data about the presence or level of liquid,
- comparing the data with a threshold,
- putting the tube into communication with the external environment to discharge the liquid as a function of the comparison.

For this last method, all the variants already descried for the previous methods and/or for the tank, are exploitable.

Another aspect of the invention is a program comprising instructions adapted to perform one of the methods described when the program is run by a microprocessor.

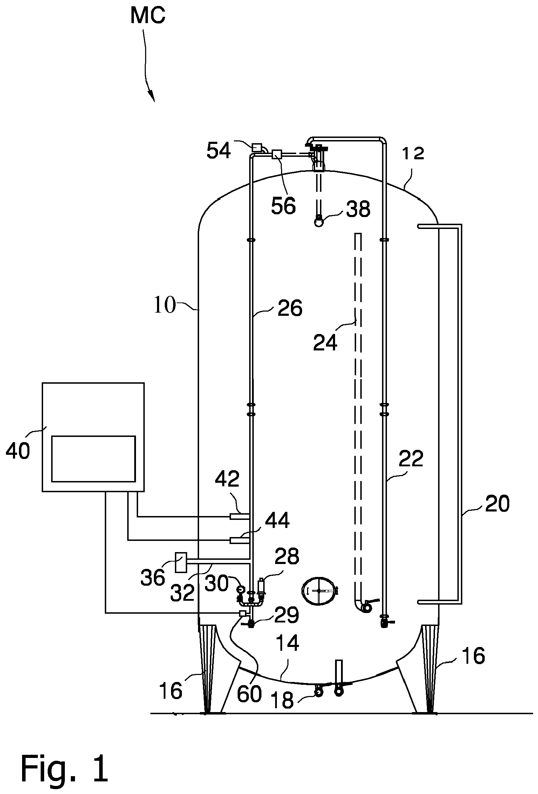

Preferred embodiments of the invention will now be described with reference to the attached drawing, wherein
- fig. 1 shows a front view of a winemaking tank equipped according to the invention.

Generally, relative terms as *vertical*, *high, top* and *bottom* are intended to refer to the tank as in use.

A winemaking tank MC comprises a closed, e.g. cylindrical, container 10 having a bottom 14 and a top 12. The bottom 14 is raised from the ground by legs 16 and comprises a drain 18 for the liquid content.

The invention can be analogously applied to horizontal-axis tanks.

Optionally on the container 10 there are mounted:
- an external level rod 20 to view from the outside the level of liquid inside the container 10. The rod 20 communicates with the bottom 14 and the top 12 of the container;
- a maximum-load tube 24, which extends vertically inside the container 10 and has a nozzle on the side of the container 10. It serves to signal the maximum level achieved during the filling phase;
- a washing tube 22 which reaches the top 12 to end on a washing bowl 38 placed inside the container 10.

On the container 10 a gas-control tube 26 is mounted, which extends vertically from the top 12 to approximately the legs 16 or the base of the container 10. The tube 26 communicates at the top with the inside of the top 12 and ends at its bottom with an (optional) manometer 30, a(n) (optional) vent valve 28 and a(n) (optional) manual discharge tap 29.

The tube 26 and/or its accessories 28, 29, 30 give operative comfort to the user of not having to climb to the top 12 for maintenance or the tank MC's control.

The tube 26 is connected to a duct 32 which arrives at a valve 36. The valve 36 is able to open if the external atmospheric pressure is greater than the one inside the container 10. Optionally the valve 36 is dual effect, that is also built to open if the pressure inside the container is greater than a threshold (e.g. 100 or 50 millibars).

The fitting 32 may also be absent, when mounting the valve 36 directly on an opening of the tube 26.

Note that the valve 36 always communicates (i.e. permanently) with the tube 26.

It should be noted that the valve 26 is easily accessible by a user who stands on the support plane of the tank MC.

Preferably, there is an electronic processing unit 40, e.g. a PLC or a microprocessor system.

The following actions relating to the command or regulation of the tank MC are meant to be carried out by the unit 40, e.g.. through appropriate program or electronic circuitry.

Preferably, a liquid sensor 44 is installed on the duct or tube 26, read and operated by the unit 40.

On the tube 26 there is preferably installed a pressure sensor 42, read and operated by the unit 40. By reading the sensor 42 the unit 40 can control the internal pressure of the container 10.

The sensor 44 has a detection point inside the duct 26, e.g. near or in correspondence to the detection point of the sensor 42 (if any). Knowing whether or not there is (a column of) liquid inside the duct 26 allows establishing:
- if inside the tube 26 the abnormality of a column of liquid occurs, which may cause incorrect reading of the pressure gauge 30 and/or the exclusion of the valve 36; and possibly
- if the value read by the sensor 42 is reliable, i.e. if the sensor 42 is correctly measuring a pressure due to fermentation gas or incorrectly due to a column of liquid present in the tube 26.

Since the tube 26 must remain empty under normal conditions, the unit 40 can detect the anomaly and alert the user, e.g. by emitting a sound or light signal.

It should be noted that the accidental emptying of the duct 26, in particularly via the tap 29, could implode the container 10 by siphoning.

Preferably, the unit 40 can automatically solve the problem of the liquid column; and is therefore connected to drive them, to:
a valve 56 mounted to isolate the duct 26 from the container 10, and
a valve 54 mounted on the top part of the duct 26 for putting it into communication with the outside environment.

A valve or drain 60 is mounted on the bottom part of the tube 26 to put it into communication with the outside environment and discharge the present fluid.

The valves 56 and 54 are optional with respect to the valve 60, being essential only to empty the tube 26. However, the valve 54 offers the advantage of speeding up the emptying or allowing it for small diameters of the tube 26, while the valve 56 prevents the venting of the container 10.

According to the equipment of the tank MC, if the unit 40 detects liquid with the sensor 44, in order to permit the drainage of the tube 26, it can automatically:
- open the valve 60, or
- open the valve 60 along with the valve 54, or
- close the valve 56 and open the valve 60, or
- close the valve 56 and open the valves 60, 54, and/or
- activate an alarm.

One can equip the tank MC with an extra safety system consisting of a rupture disc attached to the top of the tank. The rupture disc has a diaphragm designed to break if the internal pressure inside the container 10 is lower than the atmospheric pressure.

It is good that the duct 32 and the tube 26 have a diameter greater or equal than that of the valve 36's opening.

## Claims

1. Method for reducing the risk of implosion of a tank (MC) capable of fermenting a vegetable product in a pressurized environment, the tank comprising
- a tube (26) which communicates with the inside of the tank and which descends from the top (12) of the tank down to its base,
- a vacuum-breaker device (36) connected permanently to the tube in proximity of the tank's base to let external air into the tube in case of depression inside the tank;
comprising the step of detecting the presence of a column of liquid inside the tube by means of a sensor (32).

2. Method according to claim 1, wherein an electrical signal is acquired from the sensor (32), the signal is evaluated by means of an electronic circuit, and in case of presence of liquid a warning device or alarm is activated.

3. Method according to claim 1 or 2, wherein an electric signal is acquired from the sensor (32), the signal is evaluated by means of an electronic circuit, and in case of presence of liquid a discharge means (60) is activated for discharging to the outside the liquid accumulated in the tube.

4. Method according to claim 3, wherein the tube is isolated from the tank before emptying it through the discharge means.

5. Method according to claim 1 or 2 or 3 or 4, wherein the presence of liquid is detected or checked at a point in which the internal pressure of the tank is detected.

6. Tank (MC) for fermenting a vegetable product in a pressurized environment, comprising
- a pressurizable container (10),
- a tube (26) which communicates with the inside of the container and which descends from the top (12) of the container to its base,
- a vacuum-breaker device (36) permanently connected to the tube (26) in proximity of the container's base to let outside air into the tube in case of depression inside the container;
a sensor (32) for detecting the presence of a liquid column inside the tube.

7. Tank according to claim 6, comprising a processing unit (40) configured or programmed to
acquire an electrical signal from the liquid sensor to detect the presence of a column of liquid within the tube, and
drive a second device in case of presence of liquid.

8. Tank according to claim 7, wherein the processing unit is configured or programmed to acquire a data from the liquid sensor and execute a function dependent on such data, e.g. to signal an anomaly (presence of liquid) by means of an acoustic or light signal emitted by the second device.

9. Tank according to claim 7 or 8, wherein the second device comprises a warning device and/or a discharge means (60) for the tube in order to discharge from the tube the accumulated liquid.

10. Tank according to any one of the preceding claims, comprising
a first valve to isolate the duct/pipe from the top of the container, and
a second valve or means for evacuating the duct/pipe,
the processing unit being configured or programmed for closing the first valve and open the second valve or a means for evacuating when the liquid sensor detects liquid.

11. Tank according to any one of the preceding claims, wherein the vacuum-breaker device (36) is connected to the tube via a fitting (32) having equal or greater cross-section than the tube's cross-section (26).

12. Method for preventing the implosion of a tank (MC) capable of fermenting a vegetable product in a pressurized environment, with the steps of:
• acquiring from a sensor (32) an electrical signal relating to the presence or level of a liquid inside a vertical tube (26) which communicates with the top (12) of the tank and descends approximately down to its base,
• determining from the electrical signal a data about the presence or level of liquid,
• comparing the data with a threshold,
• putting the tube into communication with the external environment to discharge the liquid as a function of the comparison.
